# EUROPEAN PATENT APPLICATION

(11) **EP 4 063 857 A1**
(43) Date of publication of application: **28.09.2022**
(21) Application number: 21382254.7
(22) Date of filing: 26.03.2021
(51) Int. Cl.: G01N 33/50

(54) **DRUG DISCOVERY ASSAY TO SCREEN FOR COMPOUNDS**

(71) Applicant: Fundació Institut de Recerca Biomèdica (IRB Barcelona), 08028 Barcelona (ES); Institució Catalana de Recerca I Estudis Avançats, 08010 Barcelona (ES)
(72) Inventor: SALVATELLA GIRALT, Xavier, 08028 Barcelona (ES); BIESAGA, Mateusz, 08028 Barcelona (ES); FRIGOLÉ VIVAS, Marta, 08028 Barcelona (ES)
(74) Representative: Hoffmann Eitle

(57) **Abstract**

The present invention provides a drug discovery assay to screen for compounds capable of modulating condensates based on the ability of the said compounds to shift the condensation propensity of a studied target, preferably an intrinsically disordered protein.

## Description

### Technical field of the invention

The present invention pertains to the biomedical field, in particular the present invention provides a drug discovery assay to screen for compounds capable of modulating biomolecular condensates based on the ability of the said compounds to shift the condensation propensity of a studied target, preferably an intrinsically disordered protein.

### Background of the invention

The discovery and study of IDPs (intrinsically disordered proteins) in the late 1990s questioned the central dogma in molecular biology that structure defines function, exemplified by the "lock- and-key" model put forward by Emil Fisher in 1894. IDPs lack a defined secondary and tertiary structure. They have multiple minima in their free energy landscape and can therefore populate several distinct conformations (from fully extended to completely collapsed). Their overall structure is better defined as a dynamic ensemble of many discrete conformational states, each of which is populated with a certain statistical weight. The structure of IDPs contrasts with that of globular proteins, which have a well-defined global minimum in their free-energy landscape and their three-dimensional fold, therefore, fluctuates around one equilibrium position.

Intrinsic disorder (ID) propensity is encoded in the primary sequence of proteins. Compared with folded proteins, IDPs have an overall low mean hydrophobicity and high net charge. Protein folding is a spontaneous process. When the hydrophobic core of a protein collapses, there is a large release of water molecules, thereby increasing the entropy of the system. Once the protein is depleted of water, the enthalpy of the interactions between amino acid residues is enhanced (hydrogen bonds and van der Waals interactions). The low hydrophobic content of IDPs leads to a weakened hydrophobic effect, thereby preventing their folding into a stable globular structure.

Globular proteins are involved mostly in processes requiring a large control of the three-dimensional structure, for instance enzymatic reactions. In contrast, IDPs have prevalent roles in regulation and signalling processes and in protein-protein interaction hubs. For example, ID is widespread in transcription and transcription regulation, particularly in the activation domain of transcription factors. More recently, it has been demonstrated that IDPs are also involved in biomolecular condensation.

IDPs can: i) interact with their partners with both high specificity and low affinity, i.e. their interactions are highly specific and short-lived, with some exceptions; ii) rapidly respond to changes in the environment; iii) bind to distinct partners through single and/or multivalent interactions and iv) bind to multiple partners. In the bound state, IDPs can remain disordered or "fuzzy" or become structured. Most binding events are through short motifs embedded within larger disordered sequences (named molecular recognition features or short linear motifs, MoRFs). MoRFs are evolutionarily conserved and usually amphipathic, which allows for the gain in secondary structure when binding. To avoid promiscuous binding, IDPs are tightly regulated by post-transcriptional modifications (PTMs) and/or by ensuring appropriate protein levels during the required time. On the one hand, PTMs can change the energy landscape of IDPs, thus stabilising or destabilising secondary structures and causing a switch between disordered and ordered states. On the other hand, ensuring appropriate IDP levels is important to prevent non-specific interactions. The cellular concentration of IDPs is regulated at all steps of production from transcript clearance and translation rate to protein degradation, as exemplified by the observation that IDPs have shorter half-lives than structured proteins.

The misregulation of IDPs is associated with disease. In fact, the D2 concept (disorder in disorders) was introduced to highlight the involvement of ID in numerous conditions, including cancer, neurodegeneration, cardiovascular diseases and diabetes. For example, the disordered oncogene c-Myc and the cancer suppressor p53 have altered abundance in many types of cancers. Also, mutations or misregulation of some disordered proteins like amyloid β-peptide and α-synuclein promote aggregation in neurodegenerative conditions like Alzheimer's disease and Parkinson's disease, respectively.

IDPs are promising drug targets. They are very abundant in our proteome, are involved in many important biological functions and are over-represented in major pathologies. Up to now, most successful strategies to target IDPs have avoided the ID nature of the target. Drugs have been directed to the enzymes that regulate IDPs, the ordered domain of a protein with large ID, or the globular binding partner. Only a few strategies have aimed to drug the ID domain directly. In fact, only two compounds targeting an IDP have reached clinical trials: MSI-1436, which targets the protein tyrosine phosphatase 1B for the treatment of obesity (Trodusquemine, NCT00509132) and cardiovascular diseases (Trodusquemine, NCT00005696), and EPI-506, which targets the disordered N-terminal transactivation domain of the androgen receptor (AR-NTD) for the treatment of castration-resistant prostate cancer (Ralaniten, NCT02606123).

Therefore, there is a need to find alternative strategies to target IDPs.

On the other hand, cytoplasm and nucleoplasm are very densely packed environments. To achieve spatiotemporal control, cells localise reactions in organelles. Classical organelles are the Golgi apparatus and the Endoplasmic Reticulum. All these organelles are membrane-bound, i.e. they have a physical separation between the interior and the exterior of the organelle, and the partition of molecules is regulated by the membrane transport machinery. However, many other organelles lack a physical separation around them and yet are able to concentrate biochemical processes. Examples include the nucleoli (for ribosome biogenesis), the Cajal bodies (for assembly and or/modification of splicing machinery), the PML bodies (for transcriptional regulation and apoptosis signaling) and the stress granules (for mRNA storage upon stress).

While membrane-less compartments differ in composition, subcellular location and function, they all share the ability to concentrate biomolecules and are formed by phase transitions. For this reason, they are termed biomolecular condensates. Phase transitions have been widely studied in physics but their application in living systems is an important emerging principle in biology. Liquid-liquid phase separation (LLPS) is one particular type of phase transition and the most physiologically relevant and studied so far in the condensates field. The first evidence that membraneless organelles were liquid and formed by LLPS was reported in 2009 in a landmark paper by Dr. Clifford Brangwynne, Dr. Tony Hyman and Dr. Frank Jülicher. They characterised P granules in the germline of *Caenorhabditis elegans* and demonstrated that they behaved like liquids: they were round and could drip, wet the surface and undergo fusion events.
In the cell, membraneless organelles allow the maintenance of concentration differences without the need for energy input. The chemical potential in both phases is equal, i.e. there is no net diffusive flux between the dense and light phase, but there is constant flux of individual molecules between them.

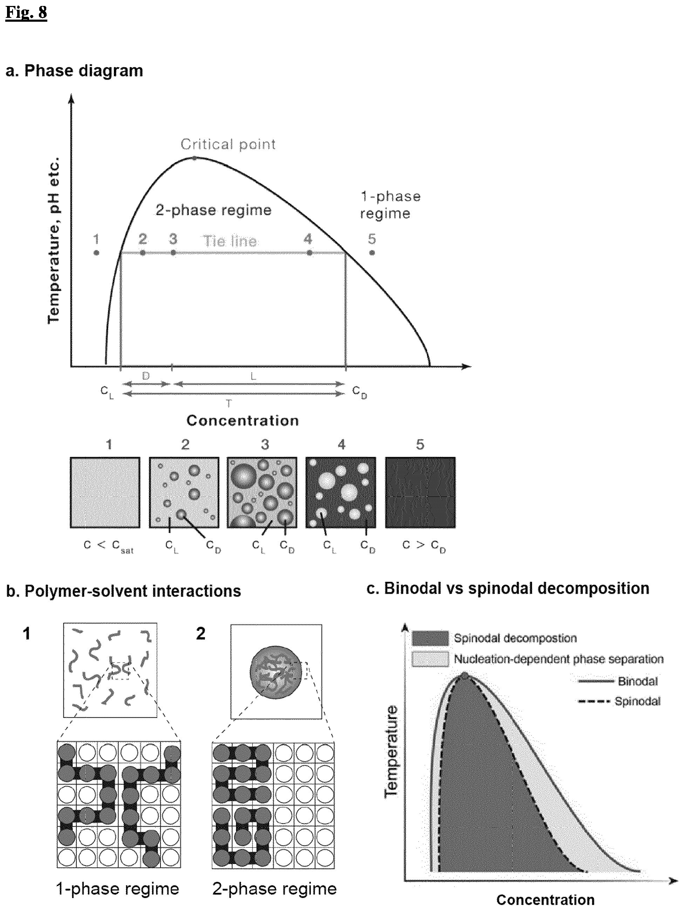

The principles of LLPS have been widely studied for polymers in soft matter physics. LLPS is the process in which a polymer that is homogeneously distributed within a solvent spontaneously demixes into two coexisting liquid phases, namely a polymer-enriched phase (dense phase) and a polymer-depleted phase (light phase) (Fig. 8a, Adapted from Alberti, S.; Gladfelter, A.; Mittag, T. Considerations and Challenges in Studying Liquid-Liquid Phase Separation and Biomolecular Condensates. Cell 2019, 176 (3), 419-434.). A negative free energy of demixing is achieved above a certain concentration (saturation concentration or Csat) with a gain in enthalpy (when polymer-polymer contacts are preferred over polymer-solvent contacts) and/or in entropy (by releasing solvent molecules from the hydration shell into the light phase) (Fig. 8b and c, Adapted from Brangwynne, C. P.; Tompa, P.; Pappu, R. V. Polymer Physics of Intracellular PhaseTransitions. Nat. Phys. 2015, 11, 899, and adapted from Posey, A. E.; Holehouse, A. S.; Pappu, R. V. Phase Separation of Intrinsically Disordered Proteins. Methods Enzymol. 2018, 611, 1-30). LLPS is extremely sensitive to changes in the physicochemical conditions of the environment. The Csat can be affected by changes in temperature, ionic strength, pH, post-translational modifications, etc... The physics behind the LLPS of polymers can be explained by the Flory-Huggins theory. However, this theory does not account for several characteristics that biopolymers have with respect to polymers (like sequence effects, the fact that they are not at thermodynamic equilibrium, etc.). For this reason, other mathematical models are needed in order to fully explain the behaviour of phase separating biopolymers.

Multivalency is the key feature for LLPS. Multivalent interactions (scaffold-scaffold and scaffold-client) allow the formation of networks, the basis for LLPS. Proteins can undergo LLPS when they contain several interaction modules with self-association affinity or affinity for a particular ligand. These interaction motifs can be structured domains connected by a disordered linker binding to a ligand or motifs (e.g. "stickers") embedded in disordered sequences ("e.g. spacers"). IDPs play a central role in LLPS as by definition they are multivalent proteins that can simultaneously interact with other molecules or with themselves. Other biomolecules that can undergo LLPS are RNA and DNA, which contain several interaction motifs with other nucleic acid molecules or proteins.

Since 2009, many studies characterising condensation have been published. Biomolecular condensates are usually heterogeneous in composition. For this reason, it is useful to apply the scaffold and client concept. Scaffold molecules are those that drive phase separation and clients are those that partition into pre-formed condensates. Biomolecular condensates participate in many cellular functions and need to be tightly controlled. Dysregulation in the assembly and disassembly of biomolecular condensates appears to be linked to various diseases including neurodegeneration, cancer and infectious diseases although the exact pathomechanisms are still unclear (e.g. whether aberrant condensate formation and dissolution is the cause of the disease or its phenotype).

Biomolecular condensates offer a new layer of regulation for IDPs. Therefore, modulating condensates with drugs is a potential opportunity to target currently undruggable proteins and contribute to drug pharmacodynamics. However, the druggability of biomolecular condensates is still an underexplored area of research.

The present invention provides a new drug discovery assay to screen for compounds capable of modulating condensates based on the ability of the said compounds to shift the condensation propensity of a studied target, preferably an intrinsically disordered protein.

### Brief description of the figures

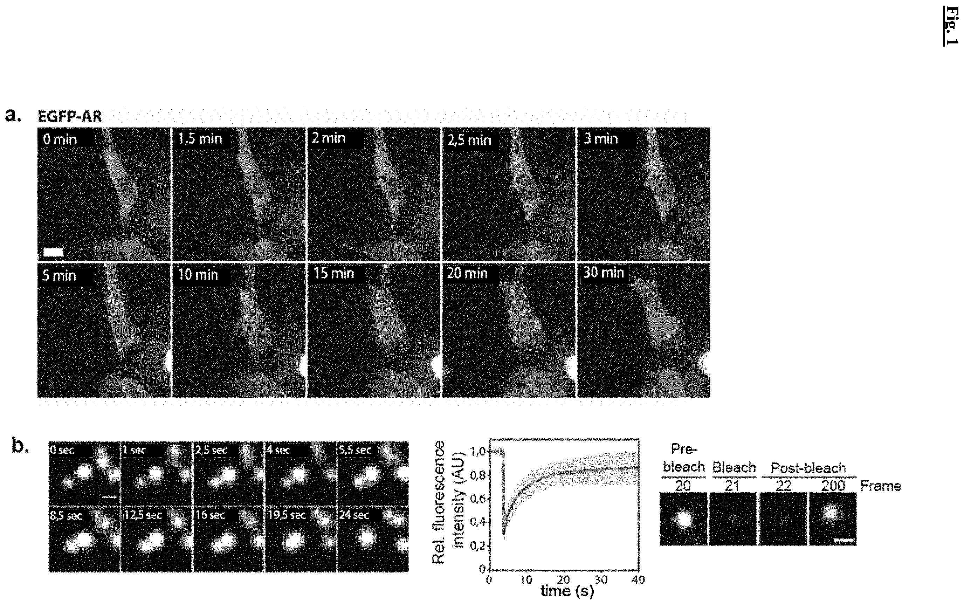

Figure 1. a) Time-resolved fluorescence microscopy of eGFP-AR upon 1 nM DHT activation in PC-3 cells (ATCC^{®} CRL-1435^{™}). b) Left: fusion event of two condensates of eGFP-AR in PC-3 cells. Right: FRAP of eGFP-AR condensates.

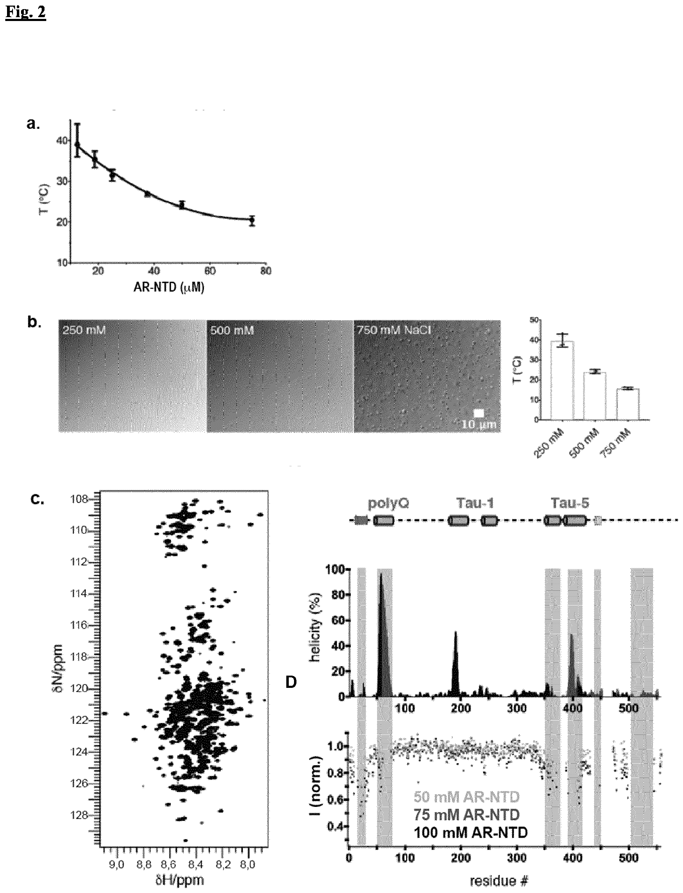

Figure 2. a) Phase diagram of AR-NTD condensation at 500 mM NaCl obtained by apparent absorbance measurements of the cloud point temperature (Tc) by means of the Turbidity Shift Assay (TSA) Left: 50 µM of AR-NTD at three ionic strengths (250 mM, 500 mM and 750 mM NaCl) observed by differential interference contrast (DIC) microscopy. Right: Tc measurement by means of the TSA. c) Left: ¹H,¹⁵N HSQC spectrum of 25 µM ¹⁵N AR-NTD. Right: plot of the residue-specific helicity using the 82D algorithm 92 and loss of peak intensity in the ¹H,¹⁵N HSQC spectrum upon AR-NTD concentration (50 µM, 75 µM and 100 µM) normalised to their intensity at 25 µM. Shaded areas indicate complete loss of signal upon concentration.

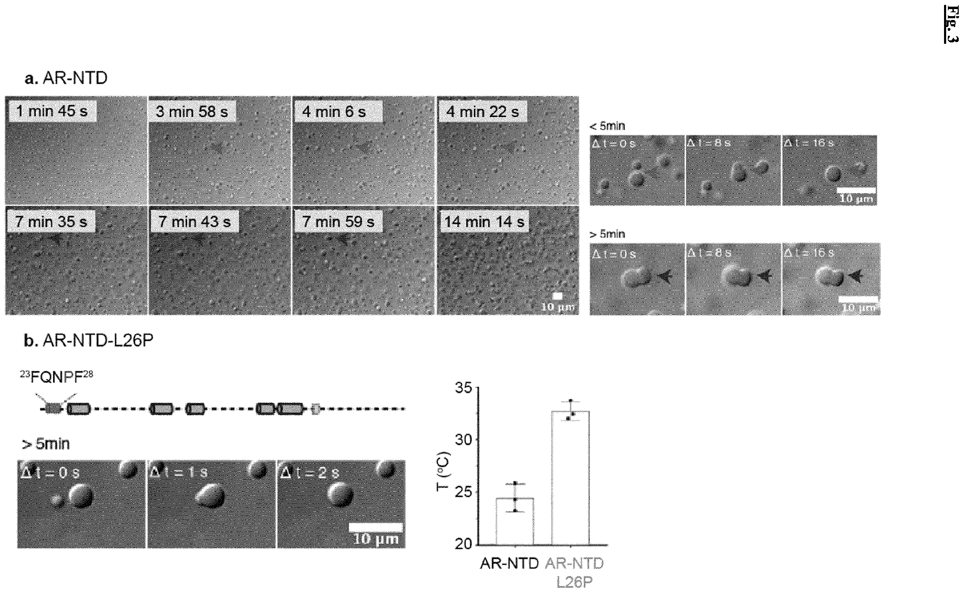

Figure 3. a) DIC images at different times of a 50 µM AR-NTD sample at 500 mM NaCl. Zoom-in on the right. b) Left: AR-NTD-L26P mutant fusion events of a sample incubated for more than 5 min. Right: Tc measurement of 50 µM AR-NTD versus 50 µM AR-NTD-L26P in 500 mM NaCl by means of the TSA assay.

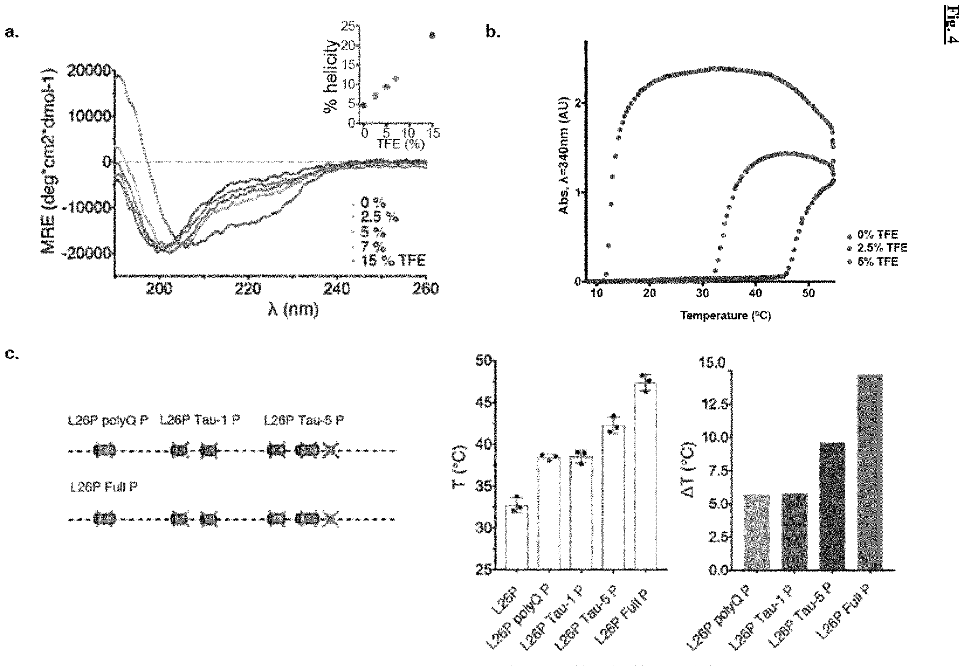

Figure 4. a) Circular dichroism (CD) spectra of AR-NTD-L26P upon TFE titration at 5 °C and calculated percentage of helicity from the CD data (insert). b) Apparent absorbance (at λ = 340 nm) measurement at increasing temperatures upon TFE titration by means of TSA. c) Left: design of helix-breaking mutants. Right: their effect on the Tc value measured by TSA.

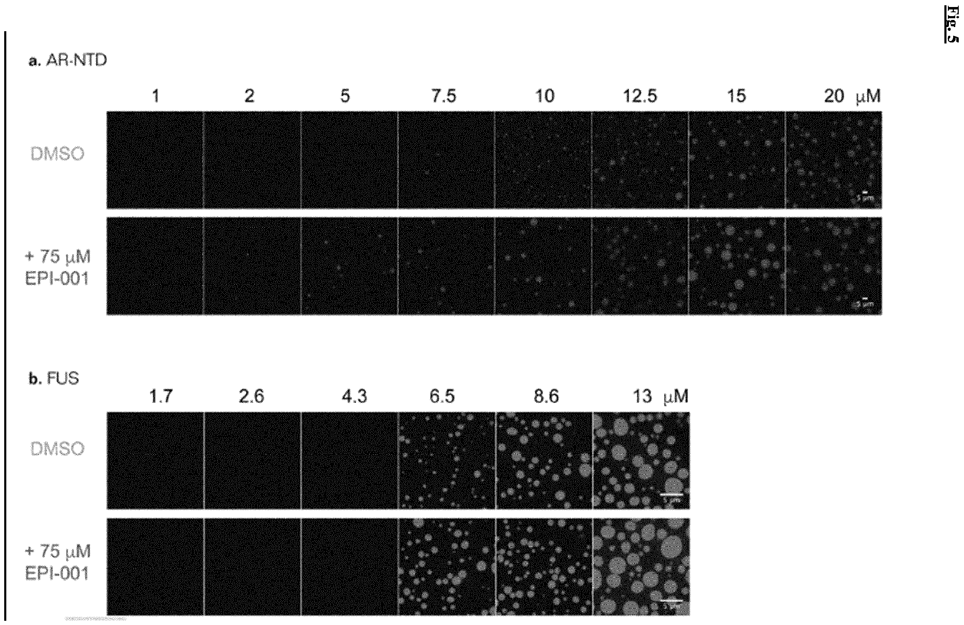

Figure 5. a) Confocal fluorescence microscopy images of the AR-NTD at different concentrations (indicated in the figure) in 20 mM Hepes 7.4, 150 mM NaCl, 10% ficoll, 2 mM TCEP buffer in the absence (top) and presence (bottom) of 75 µM EPI-001. 1% of the AR-NTD is rhodamine-labelled in all samples. Scale bar, 5 µm. b) Confocal fluorescence microscopy images of FUS-GFP at different concentrations (indicated in the figure) in 25 mM Tris-HCl 7.4, 150 mM KCl, 2.5% glycerol and 0.5 mM DTT buffer in the absence (top) and presence (bottom) of 75 µM EPI-001. Scale bar, 5 µm.

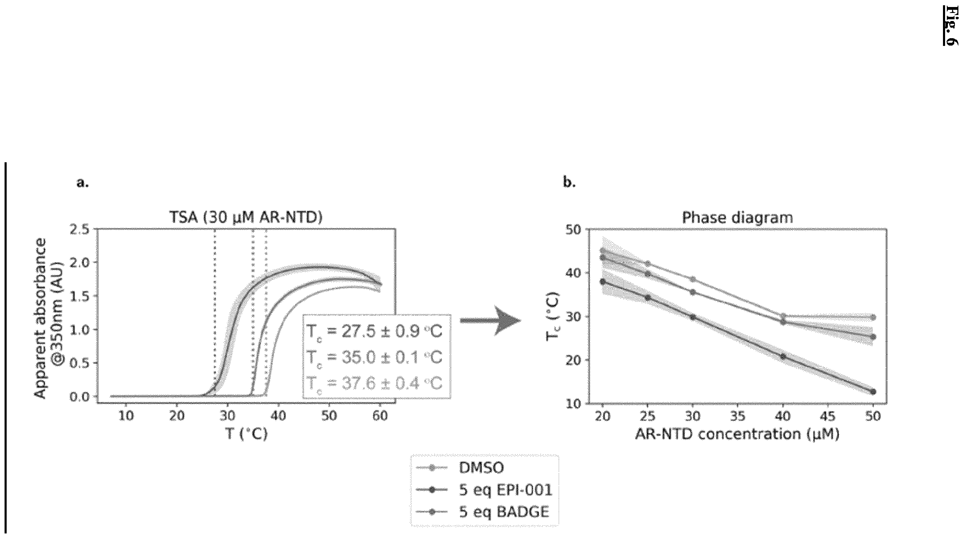

Figure 6. a) Apparent absorbance (at λ = 350 nm) measurement at increasing temperatures of 30 µM ARNTD in 20 mM Hepes 7.4, 500 mM NaCl, 2 mM TCEP buffer in the absence (grey) and presence of 5 eq of EPI-001 (red) or BADGE (green). Dashed lines indicate condensation onset and calculated Tc values are depicted in the insert. b) Tc obtained using the TSA assay of AR-NTD at different concentrations in 20 mM Hepes 7.4, 500 mM NaCl, 2 mM TCEP buffer in the absence (grey) and presence of 5 eq of EPI-001 (red) or BADGE (green). Mean values of 3 independent measurements and the standard deviation are plotted.

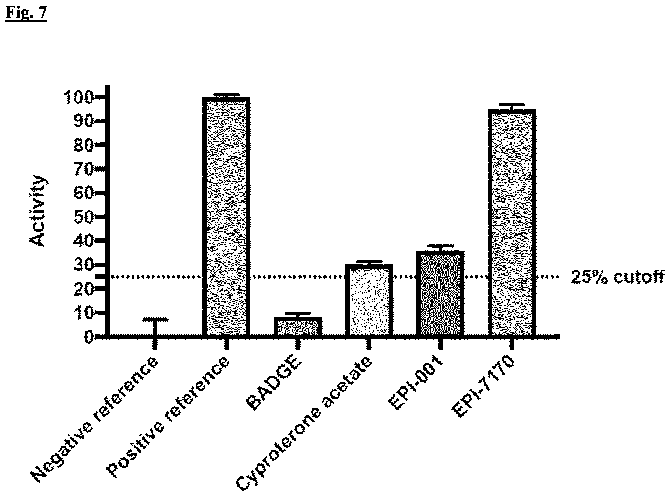

Figure 7. Results of screening for molecules using the condensation assay. First two bars show data points corresponding to the controls that are used to calculate the activity: Negative reference (DMSO), Positive reference (EPI-001 at 200 µM). The rest of the bars corresponds to the activity of four screened molecules at a screening concentration 100 µM. The activity cut-off was set at 25%, which allows to distinguish between an inactive compound BADGE and three hits: cyproterone acetate, EPI-001 and EPI-7170. Mean values of 3 measurements and the standard deviation are plotted.
Figure 8. a) The phase diagram is the representation of the one-phase and two-phase regimes depending on environmental conditions such as temperature, pH, ionic strength, etc. (black line). (1) At concentrations below Csat, the system is in the one-phase regime. (2) At any condition within the two-phase regime, the system demixes into a light phase (with c=cL) and a dense phase (with c = cD). (2-4) At increasing concentrations, only the volume fractions of the two phases change relatively to each other but cL and cD remain constant. Adapted from Alberti et al. 2019. b) (1) Below the Csat the polymer is homogeneously distributed in the solvent. (2) Above the Csat the system demixes into two phases and polymer-polymer interactions are stronger. Adapted from Brangwynne et al. 2015. c) There is a region of instability at the interface between the one-phase and two-phase regimes in which the system demixes when nucleated. At the interface, a metastable well-mixed state, albeit supersaturated, can persist even when phase separation is thermodynamically favourable. In this scenario, nucleation accelerates and determines whether phase separation takes place. Adapted from Posey et al. 2018.

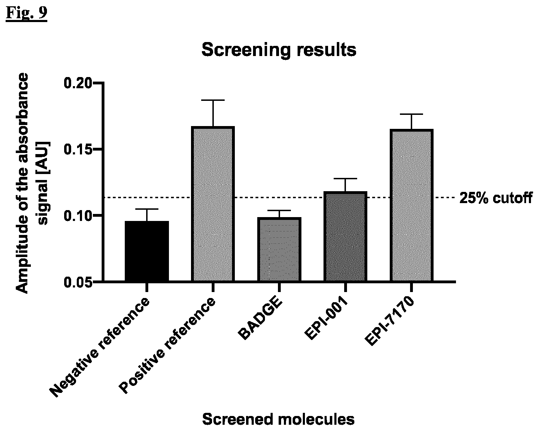

Figure 9. Results of screening for molecules using the condensation assay with amplitude of the absorbance as a parameter determining the activity of tested molecules. First two bars show data points corresponding to the controls that are used to calculate the activity: negative reference (DMSO), positive reference (EPI-001 at 200 µM). The rest of the bars corresponds to the activity of three screened molecules at a screening concentration 100 µM. The activity cut-off was set at 25%, which allows to distinguish between an inactive compound BADGE and two hits: EPI-001 and EPI-7170. Mean values of 3 measurements and the standard deviation are plotted.

### Detail description of the invention

### Definitions

As used herein and in the appended claims, the singular forms "a," "an" and "the" include plural referents unless the context clearly dictates otherwise. Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising, " those skilled in the art would understand that in some specific instances, an embodiment can be alternatively described using language "consisting essentially of" or "consisting of".

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

As used herein, the words or terms set forth below have the following definitions:
"About" means that the item, parameter or term so qualified encompasses a range of plus or minus ten percent above and below the value of the stated item, parameter or term.

As used herein, "intrinsically disordered protein" is understood as a protein that lacks a defined three-dimensional structure.

As used herein, "shifting the condensation propensity" is understood as changing a quantifiable parameter describing a property of a condensate. In particular, "shifting the condensation propensity" can mean modifying the phase diagram of a system, i.e. modifying the boundary between the regimes on such phase diagram. As an example, shift of such boundary between the regimes can be observed when, for a tested condition and a reference condition, condensation is observed at different values of particular parameters of the system, i.e. temperature, protein concentration, salt concentration or other. On the contrary, the condensation propensity is not shifted when, for a tested condition and a reference condition, condensation is observed at the same values of system parameters.

As used herein, "prior to forming condensates" is understood as a condition at which the condensation has not yet occurred, in contrast to a condition at which condensation has been detected after inducing it by changes of system parameters, e.g. change in temperature, salt concentration, pH or any other parameter that can induce condensation.

As used herein, "target" is understood as any biomolecule, non-limiting examples of which include a protein or a nucleic acid, that is involved in one or more physiological or pathological processes which can be modified upon pharmacological intervention directed at such biomolecule.

As used herein, "extra-condensate solution" is understood as a solution comprising buffer, salts, optionally reducing agents and molecular crowders with predefined salt molarity and pH, allowing to observe condensation onset within assayed temperatures.

As used herein, "spectroscopy-based measurement" is understood as measurement of energy or intensity in a beam of light or radiation of a specific wavelength. In particular, the measured parameters are based on absorption, emission, and/or scattering of electromagnetic radiation by atoms, molecules, and/or ions within the material.

As used herein, the term "significant/ly" as "significantly greater" is understood as having low probability to occur by chance. Such significance can be defined when two sets of data are compared by statistical analysis. For example, statistical term p expresses the percent chance of the result occurring by random accident. In this case, the sets of data are considered significant when p<0.05, which value correspond to 5% chance of obtaining the result by serendipity.

As used herein, "EPI-001" is understood as a small molecule that binds to AR-NTD, is characterised by *in vitro* and in cell activity against castration resistant prostate cancer and can be represented by the following structure (I):

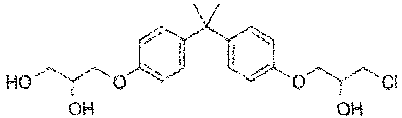

As used herein, "EPI-001 analogues" are understood as compounds included in the same family as EPI-001 bearing two aromatic rings with or without substitutions linked by a 1-atom linker (please refer to WO/2011/082488).

As used herein, "EPI-7170" is understood as a small molecule that binds to AR-NTD, is characterised by *in vitro* and *in vivo* activity against castration resistant prostate cancer and can be represented by the following structure (II):

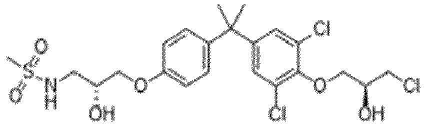

As used herein, "androgen receptor" ("AR") is understood as a molecule that is a member of the nuclear receptor family of proteins. AR binds the androgens and mediates their physiological effects by transactivation of androgen-responsive genes. The wild-type AR reference sequence corresponds to Uniprot database Accession No. P10275.

As used herein, "androgen receptor N-terminal domain" is understood as a domain of the androgen receptor localised at its N-terminus, amino acids 1-558. Androgen receptor N-terminal domain is an intrinsically disordered domain indispensable for the transactivation of AR

As used herein, "multiplex device" is understood as a device capable of processing a plurality of samples in an automatized manner. In particular, this device is capable of performing measurements of a plurality of samples without the need of intervention of a user of such device. In particular, multiplex device is a plate reader capable of processing plurality of samples located on a reading plate.

As used herein, "tc" is understood as the time measured while performing the screening assay used for drug discovery purposes that is the subject of the invention described herein at which the onset of the condensation is observed. Specifically, said tc parameter is measured from the moment when the temperature starts to be increased or decreased. In particular, tc equals to zero would account for the moment when the temperature starts to be ramped up or down.

As used herein, "onset of the condensation" is understood as a value of a parameter such as time or temperature at which condensation occurs. In particular, onset of the condensation can be determined during a measurement in which such parameters are being gradually and monotonously changed. In this case, condensation is observed after the onset of the condensation and not before.

As used herein, "Critical Temperature" is understood as the temperature at which the onset of condensation is observed for a given sample during a gradual increase or decrease of the temperature.

As used herein, "Cyproterone acetate (CPA)", is understood as an antiandrogen and progestin medication which is used in the treatment of androgen-dependent conditions like acne, excessive hair growth, early puberty, and prostate cancer, as a component of feminizing hormone therapy for transgender women, and in birth control pills.

As used herein, "significantly shifts the critical temperature of reference at which the target forms the condensates", shall be understood as an event of a change of the critical temperature of the sample relative to a reference, where such shift is characterised by a difference in critical temperature value greater than a difference that would come from a variability of the measurements. In particular, such variability is measured by statistical parameters such as standard deviation, and in this case, "significantly shifts the critical temperature of reference at which the target forms the condensates" would mean a shift of the critical temperature greater than a standard deviation of the critical temperature of the reference sample. This does not exclude other statistical parameters to be employed for this purpose.

As used herein, "amplitude of the absorbance signal of reference" is understood as a value described as the difference between the maximal absorbance value, measured after the condensation of the sample and either the background absorbance value measured prior to condensation of the sample or its approximate, such as value determined from a mathematical function to the absorbance values prior to condensation.

As used herein "constant speed" is understood as a constant change of a parameter in time. In particular, such parameter can be temperature, in which case changing temperature at a constant speed is understood as a linear change of the temperature in time.

As used herein, "N/C interaction" is understood as the interaction between amino acids belonging to N-terminal domain and C-terminal domain of androgen receptor.

### Description

The present invention provides a new drug discovery assay to screen for compounds capable of modulating condensates based on the ability of the said compounds to shift the condensation propensity of a studied target, preferably of an intrinsically disordered protein.

From the data shown in example 1, several observations led us to realise that the androgen receptor (AR) undergoes biomolecular condensation in cells and that the disordered androgen receptor N-terminal domain (AR-NTD) is sufficient to recapitulate the process *in vitro.* Since the AR-NTD is key for the condensation of the receptor and EPI-001 is known to bind to the AR-NTD, we hypothesised that the mode of action of EPI-001 involved the modulation of the ability of the AR to condensate. From previous experiments we know that EPI-001 binding enhanced the AR-NTD oligomerisation process. This is because EPI-001 promotes and stabilises those conformations prone to self-associate. If we translate these findings to the condensation field, EPI-001 could be able to lower the saturation concentration (Csat) of the AR-NTD by stabilising the condensed phase and/or the conformations prone to condensation.

To quantify the effects of EPI-001 on the AR-NTD phase separation, we implemented a new temperature-dependent turbidity shift assay (TSA), which constitutes one of the main aspects of the present invention. In particular, as illustrated in example 2 we determined whether EPI-001 had an effect on the condensation of the AR-NTD *in vitro.* We imaged the AR-NTD at increasing concentrations in conditions for phase separation (10% ficoll and 150 mM NaCl) in the absence and presence of 75 µM EPI-001 (Fig. 5). In the conditions tested without EPI-001, the AR-NTD formed droplets at around 7.5 µM. In the presence of EPI-001, the AR-NTD already demixed at 2 µM. To quantify the effects of EPI-001 on the AR-NTD phase separation, we implemented the temperature-dependent turbidity shift assay (TSA). In this assay we measure the sample turbidity as a reporter of condensation and determine the temperature at which a given sample demixes, the critical Temperature (Tc). When droplets are formed, they scatter light and cause an abrupt increase in turbidity. Therefore, theoretically values above 0 absorbance units (AU) in the TSA assay indicate condensation. In practice, the reference value before condensation is determined as the absorbance of the sample before onset of temperature ramping. Values that are above this reference value will indicate condensation. We measure turbidity as the apparent absorbance at a wavelength in which the absorption of the other components in the sample is minimal, in our case 350 nm. Addition of 5 equivalents (eq) of EPI-001 to a 30 µM AR-NTD and 500 mM NaCl sample lowered the Tc of the AR-NTD by 10 °C (Fig. 6a). This is consistent with the imaging data (Fig. 5) and consolidates the finding that EPI-001 promotes the AR-NTD condensation.

Hence, the above data shows, among other facts, that the TSA assay of the present invention constitutes a novel cell-free drug discovery assay that allows the identification of compounds capable of shifting the condensation propensity of a studied target, preferably an intrinsically disordered protein. Moreover, as shown in example 3, the ability of the TSA assay to identify compounds capable of shifting the condensation propensity of a studied target, preferably an intrinsically disordered protein, opens the possibility of finding new and improved inhibitors of such targets.

Therefore, a first aspect of the invention refers to a, preferably cell-free, drug discovery assay to screen for compounds capable of shifting the condensation propensity of a studied target, preferably an intrinsically disordered protein, the assay comprising:
a. combining a test compound and a composition comprising the target prior to forming condensates in an extra-condensate solution;
b. causing the formation of the target condensate in the presence of the test compound by a gradual and controlled increase of the temperature of the solution of a), preferably at a constant speed, such gradual and controlled increase of the temperature of the solution of a), preferably at a constant speed, is preferably performed at any speed selected from any value from 0.5 to 5°C degrees/minute, more preferably from 0.8 to 2°C degrees/minute;
c. measuring the solution of a) by any spectroscopy-based measurement, preferably by measuring the absorbance, while performing the gradual and controlled increase of the temperature according to step b), so as determining whether the test compound shifts the critical temperature of reference at which the target forms the condensates, wherein the critical temperature of reference at which the target forms the condensates is understood as the temperature in which the target forms the condensates without the presence of the test compound in the extra-condensate solution; and
selecting the test compound as a compound capable of shifting the condensation propensity of a studied target if the test compound significantly shifts the critical temperature of reference at which the target forms the condensates, or if the percentage of shift caused by the test compound in comparison to a reference shift caused by a reference compound is significantly greater, preferably greater than at least two to three standard deviations from the mean of activity values of the reference compound, preferably greater than about 20%, 25%, 30%, 35% or 40% of the shift caused by the reference compound.

In another preferred embodiment of the first aspect of the invention, the cell-free assay comprises:
a) Using a multiplex device for combining, in each well of the multiplex device, a compound from a plurality of test compounds (library of test compounds) and a composition comprising the target prior to forming condensates in an extra-condensate solution;
b) causing the formation of the target condensate in the presence of the test compound by a gradual and controlled increase of the temperature of the multiplex device, preferably at a constant speed, such gradual and controlled increase of the temperature of the solution of a), preferably at a constant speed, is preferably performed at any speed selected from any value from 0.5 to 5°C degrees/minute, more preferably from 0.8 to 2°C degrees/minute;
c) measuring the solution of a) by any spectroscopy-based measurement, preferably by measuring the absorbance, while performing the gradual and controlled increase of the temperature according to step b), so as determining whether the test compound shifts the critical temperature of reference at which the target forms the condensates, wherein the critical temperature of reference at which the target forms the condensates is understood as the temperature in which the target forms the condensates in each well without the presence of the test compound in the extra-condensate solution; and
selecting the test compound/s as those compound/s capable of shifting the condensation propensity of a studied target if the test compound/s significantly shifts the critical temperature of reference at which the target forms the condensates or if the percentage of shift caused by the test compound in comparison to a reference shift caused by a reference compound is significantly greater, preferably greater than at least two or three standard deviations from the mean of activity values of the reference compound, preferably greater than about 20%, 25%, 30%, 35% or 40% of the shift caused by the reference compound.

It is noted that as defined in the examples, the extra-condensate solution shall be understood as a solution comprising buffer, salts, optionally reducing agents and molecular crowders with predefined salt molarity and pH, allowing to observe condensation onset within assayed temperatures. Particular non-limiting examples of extra-condensate solutions are illustrated as NaP buffer with 500 mM of NaCl and 1 mM of TCEP and pH 7.4 or HEPES buffer with 150 mM of NaCl, 1 mM of TCEP and 10% (v/v) ficoll and pH 7.6.

In a preferred embodiment of the first aspect of the invention, such gradual and controlled increase of the temperature at a constant speed is preferably selected from any constant speed from 0.1 to 5°C degrees/minute, preferably from 0.3 to 3°C degrees/minute, more preferably from 0.5 to 1.5°C degrees/minute, still more preferably from 0.9 to 1.1°C degrees/minute, still more preferably the increase is of about 1°C.

In another preferred embodiment of the first aspect of the invention or of any of its preferred embodiments, the spectroscopy-based measurement is selected from the list consisting of absorbance, fluorescence, fluorescence polarization, radiometry, colorimetry, luminescence or light scattering measurements.

Another preferred embodiment of the first aspect of the invention or of any of its preferred embodiments, further comprises labelling the target in order to aid visualizing the target condensate, such as with a radioactive label, a colorimetric label, enzyme fragments, a fluorescent label, or others

In another preferred embodiment of the first aspect of the invention or of any of its preferred embodiments, the composition comprises two or more biomolecules/proteins, from which at least one is a target.

In another preferred embodiment of the first aspect of the invention or of any of its preferred embodiments, the amount of the test compound/s is further determined by, for example, mass spectrometry, liquid chromatography, and/or ultraviolet-visible spectrophotometry.

In another preferred embodiment of the first aspect of the invention or of any of its preferred embodiments, the target is or is comprised in a protein selected from the list consisting of TAR DNA-binding protein 43 (TDP-43), tau protein, members of myc family of transcription factors, fused in sarcoma protein (FUS), hnRNPAI, PGL-3, huntingtin protein, androgen receptor (AR) and other members of nuclear receptor family of proteins or any fragments thereof. Preferably, the target is the N-terminal domain of the androgen receptor (AR-NTD).

In another preferred embodiment of the first aspect of the invention or of any of its preferred embodiments, the target is the N-terminal domain of the androgen receptor (AR-NTD) and the reference compound is compound EPI-001 or any analogues thereof. Furthermore, in this context of the invention, the extra-condensate solution comprises NaCl and has a pH between 7.0 and 7.8, and optionally a cryopreserving agent, and the Critical Temperature of AR-NTD is detected within an assay window between 30°C and 49°C.

In an alternative embodiment to the first aspect of the invention or to any of its preferred embodiments, the, preferably cell-free, drug discovery assay to screen for compounds capable of shifting the condensation propensity of a studied target, preferably of an intrinsically disordered protein, comprises measuring the solution of a) of the first aspect of the invention, by any spectroscopy-based measurement, preferably by measuring the absorbance, while performing the gradual and controlled increase of the temperature according to step b) of the first aspect of the invention, so as determining whether the test compound shifts the tc value of reference at which the target forms the condensates, wherein the tc value of reference is the time between the beginning of the gradual and controlled increase of the temperature of the solution of a) and the onset of condensation in conditions where the target forms the condensates without the presence of the test compound in the extra-condensate solution; and
selecting the test compound as a compound capable of shifting the condensation propensity of a studied target if the test compound significantly shifts the tc value of reference at which the target forms the condensates or if the percentage of shift caused by the test compound in comparison to a reference shift caused by a reference compound is significantly greater, preferably greater than at least two or three standard deviations from the mean of activity values of the reference compound, preferably greater than about 20%, 25%, 30%, 35% or 40% of the shift caused by the reference compound.

Preferably, the cell-free drug discovery assay to screen for compounds capable of shifting the condensation propensity of a studied target, preferably an intrinsically disordered protein, comprises measuring the absorbance of each of the wells in a multiplex device, while performing the gradual and controlled increase of the temperature according to step b) of the first aspect of the invention, so as determining whether one or more of the test compounds shift the tc value of reference at which the target forms the condensates, wherein the tc value of reference is the time between the beginning of the gradual and controlled increase of the temperature of the multiplex device and the onset of condensation in each well in conditions where the target forms the condensates without the presence of the test compound in the extra-condensate solution; and
selecting the test compound/s as those compound/s capable of shifting the condensation propensity of a studied target if the test compound/s significantly shifts the tc value of reference at which the target forms the condensates or if the percentage of shift caused by the test compound in comparison to a reference shift caused by a reference is significantly greater, preferably greater than at least two or three standard deviations from the mean of activity values of the reference compound, preferably greater than 20%, 25%, 30%, 35% or 40% of the shift caused by the reference compound.

That is, in this alternative embodiment, the tc value is measured instead of the critical temperature as reflected in the initial embodiments of the first aspect of the invention.

In another alternative embodiment to the first aspect of the invention, the cell-free drug discovery assay to screen for compounds capable of shifting the condensation propensity of a studied target, preferably an intrinsically disordered protein, comprises measuring the solution of a) of the first aspect of the invention, by any spectroscopy-based measurement, preferably by measuring the absorbance, while performing the gradual and controlled increase of the temperature according to step b) of the first aspect of the invention, so as determining whether the test compound shifts the amplitude of the absorbance signal of reference at which the target forms the condensates, wherein the amplitude of the absorbance signal of reference is a value described as the difference between the maximal absorbance value measured after the condensation of the sample and either the background absorbance value measured prior to condensation of the sample or its approximate, such as value determined by fitting a mathematical function to the absorbance values prior to condensation; and selecting the test compound as a compound capable of shifting the condensation propensity of a studied target if the test compound significantly shifts the amplitude of the absorbance signal of reference at which the target forms the condensates or if the percentage of shift caused by the test compound in comparison to a reference shift caused by a reference compound is significantly greater, preferably greater than at least two or three standard deviations from the mean of activity values of the reference compound, preferably greater than about 20%, 25%, 30%, 35% or 40% of the shift caused by the reference compound. Please note that, with reference to this aspect of the invention, the inventors have shown that this parameter could also be used to select hits (see figure 9).

That is, in this alternative embodiment, the amplitude of the absorbance signal of reference is measured instead of the critical temperature as reflected in the initial embodiments of the first aspect of the invention.

In another alternative embodiment to the first aspect of the invention or to any of its preferred or alternative embodiments, the cell-free drug discovery assay to screen for compounds capable of shifting the condensation propensity of a studied target, preferably an intrinsically disordered protein, comprises measuring the solution of a) of the first aspect of the invention, by any spectroscopy-based measurement, preferably by measuring the absorbance, but instead of performing the gradual and controlled increase of the temperature according to step b) of the first aspect of the invention or of any of its preferred or alternative embodiments, such step is performed by the gradual and controlled increase of a salt in the sample or by the gradual and controlled increase of the pH of the sample or by the gradual and controlled increase of molecular crowders in the sample. That is, in this alternative embodiment, instead of performing the gradual and controlled increase of the temperature according to step b) of the first aspect of the invention or of any of its preferred or alternative embodiments, such step is performed by the gradual and controlled increase of a salt in the sample or by the gradual and controlled increase of the pH of the sample or by the gradual and controlled increase of molecular crowders in the sample.

A second aspect of the invention refers to a method for ranking or classifying a plurality of test compounds for their ability to shift the condensation propensity of a studied target, the method comprising:
a. Determining the percentage of shift caused by each compound in comparison to a reference shift by using the method of the first aspect of the invention or of any of the preferred or alternative embodiments of the first aspect of the invention; and
b. Ranking or classifying the plurality of test compounds in accordance with the percentage of shift caused by each compound.

Preferably, the method of the second aspect comprises determining the activity of the compounds in a dose-response manner. In particular, the ability to determine additional parameters such as EC50.

A third aspect of the invention refers to a method of selecting or identifying one or more test compounds for their ability to shift the condensation propensity of a studied target, the method comprising:
a. Determining the percentage of shift caused by each compound in comparison to a reference shift by using the method of the first aspect of the invention or of any of the preferred or alternative embodiments of the first aspect of the invention; and
b. Identifying the compound with the highest shifts.

A fourth aspect of the invention refers to a method of preparing one or more test compounds for their ability to shift the condensation propensity of a studied target, the method comprising, preparing a composition comprising said one or more test compounds at a concentration at which the compound is capable of shifting the condensation propensity of a studied target according to the data provided by the method of identifying one or more test compounds of the third aspect of the invention.

The following examples merely illustrate the present invention but do not limit the same.

### Examples

### Example 1. Androgen receptor undergoes biomolecular condensation

During the course of this invention, we uncovered that the androgen receptor N-terminal domain (AR-NTD) underwent biomolecular condensation *in vitro* and in cells.

In cells, androgen receptor (AR) underwent condensation upon hormone (dihydrotestosterone, DHT) binding (Fig. 1a). Overexpressed eGFP-AR was kept soluble in the cytoplasm in several cell lines. Addition of 1 nM DHT nucleated eGFP-AR condensation in the cytosol. AR condensates had liquid-like properties as they were spherical, fused and relaxed into a sphere within seconds and were highly dynamic according to fluorescence-recovery after photobleaching (FRAP) experiments (Fig. 1b). 30-60 min after hormone addition, the eGFP-AR translocated to the nucleus, in agreement with the AR classical pathway. The fact that i) the AR underwent condensation upon DHT addition and ii) that DHT addition caused the interaction between the ²³FQNLF²⁷ and the AR ligand-binding domain (AR-LBD) suggested that the N/C interaction plays a role in the AR condensation.

Purified AR-NTD (AR1-558) underwent LCST type phase separation in vitro (Fig. 2a). AR-NTD condensation is driven by hydrophobic and aromatic interactions. This was determined by mapping the dependence of the AR-NTD condensation to the ionic strength (NaCl concentration) of the buffer (Fig. 2b). To determine which regions of the AR-NTD are involved in the oligomerisation and phase separation process, solution nuclear magnetic resonance (NMR) spectroscopy of the light phase was used (Fig. 2c). The ¹H,¹⁵N HSQC spectrum of the AR-NTD was acquired at increasing protein concentrations. The regions of the AR-NTD with a concentration-dependent decrease in signal intensity had an increased ¹⁵N transverse relaxation rate. This indicated that concentration led to more transient inter-residue long-range interactions. Particularly, the most affected regions corresponded to the following motifs: the ²³FQNLF²⁷ motif, the helical ⁵⁴LLLL⁵⁷ sequence preceding the polyQ tract, the two partially helical motifs in Tau-5 region (R1 and R2) and the highly hydrophobic and aromatic region after the polyG tract. The polyQ tract and the R3 helical region in the Tau-5 could not be detected.

However, we observed that the AR-NTD droplets underwent rapid maturation in vitro (within minutes) as fusion events were arrested and "worm-like" strings of droplets formed (Fig. 3a). We demonstrated that this liquid-to-solid transition was due to the aggregation prone ²³FQNLF²⁷ motif. Indeed, when ²³FQNLF²⁷ fiber formation was perturbed by a Leu to Pro mutation (²³FQNPF²⁷), the AR-NTD-L26P droplets maintained a liquid-character throughout the duration of the experiments (Fig. 3b). The mutation also affected the condensation propensity of the AR-NTD, as the AR-NTD-L26P mutant had lower propensity to phase separate (Fig. 3c). Finally, since most of the sticky motifs in the AR-NTD that had been determined by NMR were predicted to be partially helical, we investigated the importance of condensation for the secondary structure propensity of these motifs. This was motivated by the hypothesis that the dry environment in the AR-NTD condensates promoted a helical gain in the motifs important for the AR function and its protein-protein interactions. Indeed, addition of 2,2,2-trifluoroethanol (TFE) to the AR-NTD-L26P increased its helical population (Fig. 4a) and its propensity to condensate (Fig. 4b). Moreover, additional helix-breaking mutations in the AR-NTD-L26P helices resulted in a decreased propensity to phase separate.

Interestingly, perturbing the three helical motifs in the Tau-5 (R1, R2 and R3) had the largest effect on condensation, suggesting a substantial contribution of the helicity in the Tau-5 region to the condensation of the AR-NTD (Fig. 4c). These observations led to a "folding upon condensation" model in which condensation would facilitate recruitment of the transcription machinery and co-activators to the AR transcriptional condensates.

From the above data, several observations led us to realise that the androgen receptor (AR) undergoes biomolecular condensation in cells and that the disordered androgen receptor N-terminal domain (AR-NTD) is sufficient to recapitulate the process *in vitro.* Since the AR-NTD is key for the condensation of the receptor and EPI-001 is known to bind to the AR-NTD, we hypothesised that the mode of action of EPI-001 involved the modulation of the ability of the AR to condensate. From previous experiments we know that EPI-001 binding enhanced the AR-NTD oligomerisation process. This is because EPI-001 promotes and stabilises those conformations prone to self-associate. If we translate these findings to the condensation field, EPI-001 could be able to lower the saturation concentration (Csat) of the AR-NTD by stabilising the condensed phase and/or the conformations prone to condensation.

To quantify the effects of EPI-001 on the AR-NTD phase separation, we implemented a new temperature-dependent turbidity shift assay (TSA), which constitutes the main object of the present invention. This assay is fully described in example 2 below.

### Example 2. In vitro modulation of the AR-NTD condensation by EPI-001

- EPI-001 reversible binding promotes condensation of the AR-NTD

First, we determined whether EPI-001 had an effect on the condensation of the AR-NTD in vitro. We imaged the AR-NTD at increasing concentrations in conditions for phase separation (10% ficoll and 150 mM NaCl) in the absence and presence of 75 µM EPI-001 (Fig. 5). In the conditions tested without EPI-001, the AR-NTD formed droplets at around 7.5 µM. In the presence of EPI-001, the AR-NTD already demixed at 2 µM. This implies that EPI-001 promotes the condensation of the AR-NTD. To evaluate whether EPI-001 could modulate the condensation of any protein of interest, we run the same experiment using as a model the Fused in Sarcoma (FUS) protein. We observed that EPI-001 did not influence FUS ability to condensate (Fig. 5b).

To quantify the effects of EPI-001 on the AR-NTD phase separation, we implemented a temperature-dependent turbidity shift assay (TSA). In this assay we measure the sample turbidity as a reporter of condensation and determine the temperature at which a given sample demixes, the Critical Temperature. When droplets are formed, they scatter light and cause an abrupt increase in turbidity. Therefore, values above 0 absorbance units (AU) in the TSA assay indicate condensation. We measure turbidity as the apparent absorbance at a wavelength in which the absorption of the other components in the sample is minimal, in our case 350 nm. Addition of 5 equivalents (eq) of EPI-001 to a 30 µM AR-NTD and 500 mM NaCl sample lowered the Tc of the AR-NTD by 10 °C (Fig. 5a). This is consistent with the imaging data (Fig. 5) and consolidates the finding that EPI-001 promotes the AR-NTD condensation. In contrast, the inactive analogue BADGE was only able to lower the AR-NTD Tc by 3 °C (Fig. 6). We run the TSA assay at various protein concentrations to build the left-arm of the phase diagram (Fig. 6b). We observed that EPI-001, but not BADGE, largely enhanced the AR-NTD condensation at all concentrations tested.

Interestingly, in the context of another work, Posey et al. used the polyphasic linkage framework to describe the influence of ligands to the phase separation of a protein. Translating their conclusions to our case study, the fact that EPI-001 lowers the AR-NTD Tc is equivalent to establishing that EPI-001 preferentially partitions into the condensed phase of the AR-NTD. If the compound would interact with both phases equally, the Tc would not be affected, as in the case of BADGE.

This example shows, among other facts, that the TSA assay constitutes a novel cell-free drug discovery assay that allows the identification of compounds capable of shifting the condensation propensity of a studied target, preferably an intrinsically disordered protein.

### Example 3. In vitro modulation of the AR-NTD condensation by EPI-001 analogues

The newly acquired knowledge on the ability of EPI-001 to enhance AR condensation along with the ability of the TSA assay to identify compounds capable of shifting the condensation propensity of a studied target, preferably an intrinsically disordered protein, opens the possibility of finding new and improved inhibitors that target the androgen receptor N-terminal domain (AR-NTD) for the treatment of castration-resistant prostate cancer (CRPC). With this aim, we have further tested the TSA assay of the present invention to determine whether molecules known to be useful in the treatment of androgen-dependent conditions such as cyproterone acetate or EPI-001 analogues such as EPI-7170, could be identified as inhibitors that target the androgen receptor N-terminal domain (AR-NTD) for the treatment of castration-resistant prostate cancer (CRPC) by using the TSA assay of the present invention.

In this sense, in figure 7 the first two bars show data points corresponding to the controls that are used to calculate the activity:
- Negative reference - DMSO
- Positive reference - EPI-001 at 200 µM

The rest of the bars correspond to activity of four screened molecules at a screening concentration 100 µM. The activity cut-off was set at 25%, which allows to distinguish a non-hit molecule BADGE and three hits: cyproterone acetate, EPI-001 and EPI-7170.

Briefly, in order to quantitatively rank the tested compounds with regards to their efficacy to enhance the AR-NTD condensation in vitro and compare it to that of EPI-001, we measured the cloud point temperature (Tc) of each of the samples at constant protein concentration (100 µM). Then, we calculated the percentage of shift caused by the compound in comparison to the reference shift, the one caused by 200 µM EPI-001. Next, we plotted the normalised shift versus the compound concentration.

In summary, this example demonstrates the ability of the TSA assay to identify compounds capable of shifting the condensation propensity of a studied target, preferably an intrinsically disordered protein, opening the possibility of finding new and improved inhibitors that target the androgen receptor N-terminal domain (AR-NTD) for the treatment of castration-resistant prostate cancer (CRPC).

## Claims

1. A drug discovery assay to screen for compounds capable of shifting the condensation propensity of a studied target, preferably an intrinsically disordered protein, the assay comprising:
a. combining a test compound and a composition comprising the target prior to forming condensates in an extra-condensate solution;
b. causing the formation of the target condensate in the presence of the test compound by a gradual and controlled increase of the temperature of the solution of a), wherein such gradual and controlled increase of the temperature of the solution of a) is performed at any speed selected from any value from 0.5 to 5°C degrees/minute, more preferably from 0.8 to 2°C degrees/minute;
c. measuring the solution of a) by any spectroscopy-based measurement, preferably by measuring the absorbance, while performing the gradual and controlled increase of the temperature according to step b), so as determining whether the test compound shifts the critical temperature of reference at which the target forms the condensates, wherein the critical temperature of reference at which the target forms the condensates is understood as the temperature in which the target forms the condensates without the presence of the test compound in the extra-condensate solution; and
selecting the test compound as a compound capable of shifting the condensation propensity of a studied target if the test compound significantly shifts the critical temperature of reference at which the target forms the condensates, or if the percentage of shift caused by the test compound in comparison to a reference shift caused by a reference compound is significantly greater, preferably greater than at least two or three standard deviations from the mean of activity values of the reference compound, preferably greater than 20%, 25%, 30%, 35% or 40% of the shift caused by the reference compound.

2. The cell-free assay of claim 1, wherein the assay comprises:
a. Using a multiplex device for combining, in each well of the multiplex device, a compound from a plurality of test compounds (library of test compounds) and a composition comprising the target prior to forming condensates in an extra-condensate solution;
b. causing the formation of the target condensate in the presence of the test compound by a gradual and controlled increase of the temperature of the multiplex device, wherein such gradual and controlled increase of the temperature of the solution of a) is performed at any speed selected from any value from 0.5 to 5°C degrees/minute, more preferably from 0.8 to 2°C degrees/minute;
c. measuring the solution of a) by any spectroscopy-based measurement, preferably by measuring the absorbance, while performing the gradual and controlled increase of the temperature according to step b), so as determining whether the test compound shifts the critical temperature of reference at which the target forms the condensates, wherein the critical temperature of reference at which the target forms the condensates is understood as the temperature in which the target forms the condensates in each well without the presence of the test compound in the extra-condensate solution; and
selecting the test compound/s as those compound/s capable of shifting the condensation propensity of a studied target if the test compound/s significantly shifts the critical temperature of reference at which the target forms the condensates or if the percentage of shift caused by the test compound in comparison to a reference shift caused by a reference compound is significantly greater, preferably greater than at least two or three standard deviations from the mean of activity values of the reference compound, preferably greater than 20%, 25%, 30%, 35% or 40% of the shift caused by the reference compound.

3. A cell-free drug discovery assay to screen for compounds capable of shifting the condensation propensity of a studied target, preferably an intrinsically disordered protein, the assay comprising:
a. combining a test compound and a composition comprising the target prior to forming condensates in an extra-condensate solution;
b. causing the formation of the target condensate in the presence of the test compound by a gradual and controlled increase of the temperature of the solution of a), wherein such gradual and controlled increase of the temperature of the solution of a) is performed at any speed selected from any value from 0.5 to 5°C degrees/minute, more preferably from 0.8 to 2°C degrees/minute;
c. measuring the solution of a) by any spectroscopy-based measurement, preferably by measuring the absorbance, while performing the gradual and controlled increase of the temperature according to step b), so as determining whether the test compound shifts the tc value of reference at which the target forms the condensates, wherein the tc value of reference is the time between the beginning of the gradual and controlled increase of the temperature of the solution of a) and the onset of condensation in conditions where the target forms the condensates without the presence of the test compound in the extra-condensate solution; and
selecting the test compound as a compound capable of shifting the condensation propensity of a studied target if the test compound significantly shifts the tc value of reference at which the target forms the condensates or if the percentage of shift caused by the test compound in comparison to a reference shift caused by a reference compound is significantly greater, preferably greater than at least two or three standard deviations from the mean of activity values of the reference compound, preferably greater than 20%, 25%, 30%, 35% or 40% of the shift caused by the reference compound.

4. The cell-free assay of claim 1, wherein the assay comprises:
a. Using a multiplex device for combining, in each well of the multiplex device, a compound from a plurality of test compounds (library of test compounds) and a composition comprising the target prior to forming condensates in an extra-condensate solution;
b. causing the formation of the target condensate in the presence of the test compound by a gradual and controlled increase of the temperature of the multiplex device, wherein such gradual and controlled increase of the temperature of the solution of a) is performed at any speed selected from any value from 0.5 to 5°C degrees/minute, more preferably from 0.8 to 2°C degrees/minute;
c. measuring the absorbance of each of the wells of the multiplex device, while performing the gradual and controlled increase of the temperature according to step b), so as determining whether the test compound shifts the tc value of reference at which the target forms the condensates, wherein the tc value of reference is the time between the beginning of the gradual and controlled increase of the temperature of the multiplex device and the onset of condensation in each well in conditions where the target forms the condensates without the presence of the test compound in the extra-condensate solution; and
selecting the test compound/s as those compound/s capable of shifting the condensation propensity of a studied target if the test compound/s significantly shifts the tc value of reference at which the target forms the condensates or if the percentage of shift caused by the test compound in comparison to a reference shift caused by a reference is significantly greater, preferably greater than at least two or three standard deviations from the mean of activity values of the reference compound, preferably greater than 20%, 25%, 30%, 35% or 40% of the shift caused by the reference compound.

5. The cell free assay of any of claims 1 to 5, wherein the extra-condensate solution is **characterized by** having a buffer, in particular with a pH and a salt concentration, that allows detection of the critical temperature at which the onset of condensation of the target molecules occurs, within an assay window of between 30°C and 49°C.

6. The cell free assay of any of the precedent claims, further comprising labelling the target in order to visualize the target condensate, such as with a radioactive label, a colorimetric label, or a fluorescent label.

7. The cell free assay of any of the precedent claims, wherein the composition comprises two or more targets.

8. The cell free assay of any of the precedent claims, wherein the amount of the test compound/s is determined by mass spectrometry, liquid chromatography, and/or ultraviolet-visible spectrophotometry.

9. The cell free assay of any of claims 1 to 8, wherein the target comprises a protein selected from the list consisting of TAR DNA-binding protein 43 (TDP-43), tau protein, fused in sarcoma protein (FUS), hnRNPAI, PGL-3, huntingtin protein and androgen receptor (AR) or any fragments thereof, including the N-terminal domain of the androgen receptor (AR-NTD).

10. The cell free assay of claim 9, wherein the target is the N-terminal domain of the androgen receptor (AR-NTD).

11. The cell free assay of claim 10, wherein the target is the N-terminal domain of the androgen receptor (AR-NTD) and the reference compound is compound EPI-001.

12. The cell free assay of claim 11, wherein the extra-condensate solution comprises NaCl and has a pH between 7.0 and 7.8, and optionally a cryopreserving agent, and the Tc of AR-NTD is detected within the assay window between 30°C and 49°C.

13. A method for ranking or classifying a plurality of test compounds for their ability to shift the condensation propensity of a studied target, the method comprising:
a. Determining the percentage of shift caused by each compound in comparison to a reference shift by using the method of any of claims 1 to 12; and
b. Ranking or classifying the plurality of test compounds in accordance with the percentage of shift caused by each compound.

14. A method of selecting or identifying one or more test compounds for their ability to shift the condensation propensity of a studied target, the method comprising:
a. Determining the percentage of shift caused by each compound in comparison to a reference shift by using the method of any of claims 1 to 12; and
b. Identifying the compound/ with the highest shifts.

15. A method of preparing one or more test compounds for their ability to shift the condensation propensity of a studied target, the method comprising, preparing a composition comprising said one or more test compounds at a concentration at which the compound is capable of shifting the condensation propensity of a studied target according to the data provided by the method of identifying one or more test compounds of claim 11.
